(19) **Europäisches Patentamt / European Patent Office / Office européen des brevets**

(11) **EP 1 972 346 B1**

(12) # EUROPEAN PATENT SPECIFICATION

(45) Date of publication and mention
of the grant of the patent:
**14.10.2015 Bulletin 2015/42**

(51) Int Cl.:
**A61K 38/16** *(2006.01)*    **A23L 1/29** *(2006.01)*

(21) Application number: **05825242.0**

(22) Date of filing: **30.11.2005**

(86) International application number:
**PCT/ES2005/000655**

(87) International publication number:
**WO 2007/063142 (07.06.2007 Gazette 2007/23)**

(54) **PROTEIN MIXTURE AND USE THEREOF IN THE PREPARATION OF A PRODUCT THAT IS INTENDED FOR ORAL OR ENTERAL FOOD**

PROTEINMISCHUNG UND IHRE VERWENDUNG BEI DER HERSTELLUNG EINES PRODUKTS ZUR VERWENDUNG ALS ORALES ODER ENTERALES NAHRUNGSMITTEL

MELANGE DE PROTEINES UTILISE POUR PREPARER UN PRODUIT DESTINE A L'ALIMENTATION ORALE OU ENTERALE

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**24.09.2008 Bulletin 2008/39**

(73) Proprietor: **Vegenat, S.A.**
**06184 Pueblonuevo del Guadiana, Badajoz (ES)**

(72) Inventors:
• **GIL HERNÁNDEZ, Angel**
**E-6011 Badajoz (ES)**
• **MARTÍNEZ DE VICTORIA MUÑÕZ, Emilio**
**E-06011 Badajoz (ES)**
• **SAN ROMÁN PAIS, Paloma**
**E-06011 Badajoz (ES)**

(74) Representative: **Gil-Vega, Victor**
**Corazón de Maria, 6**
**28002 Madrid (ES)**

(56) References cited:
| | |
|---|---|
| **EP-A2- 0 626 175** | **EP-A2- 0 626 176** |
| **ES-T3- 2 142 842** | **US-A- 5 504 072** |
| **US-A- 5 514 656** | **US-A- 5 766 621** |
| **US-A1- 2001 043 958** | **US-A1- 2003 104 033** |
| **US-A1- 2004 202 765** | **US-B1- 6 241 996** |

• **DATABASE WPI Week 200601, Derwent Publications Ltd., London, GB; AN 2006-000101, XP003014739 & BR PI0 401 405 A (PIMENTEL GUIMARAES, A.R.) 08 November 2005**

**Description**

This invention relates to a novel protein mixture and the use thereof in the preparation of a food product intended for oral or enteral nutrition.

Whether with oral or enteral administration products, there is a large number of social groups that require coverage of their nourishing needs with this type of nutritive products to avoid malnutrition. This malnourishment is more frequent in older population, since even when they do not suffer from any specific pathology associated to nutrition, this group presents factors closely related to the same, for example decreased number of papillae, lack of teeth that renders mastication difficult, less secretion of saliva and decreased metabolic rate and together with it, a decreased need to eat.

This type of artificial nutrition is also used in several pathological conditions, for example people affected by cerebrovascular accidents, multiple sclerosis, Parkinson's or Alzheimer's diseases, people suffering from mouth or throat cancer or esophageal damage, in general people showing hypermetabolic states with different etiologies (burns, traumas, surgery), chronic illnesses or people who have been subject to prolonged periods of reduced oral intake. All these possible situations have to be taken into account in the case of inpatients, where nutrition control is relatively easier, as well as in the case of outpatients where homecare is in charge of health staff or the patient's relatives or even himself/herself.

There are several formulations intended for oral or enteral nutrition, products made of a mixture of nutrients classified within the legal framework as "dietetic products for specific nutritional purposes". These may consist of nutritionally complete formulae, which contain an adequate quantity of each and all nutrients necessary for a balanced nutritional state, supplements, which are nutritionally incomplete and supplement an adequate oral diet, and finally modules, which contain one specific nutrient. In the case of oral administration formulae, additives such as flavours and aromas are added to improve palatability. Complete formula products, where the nourishing formula contains proteins, carbohydrates, fat, fibre and micro nutrients such as the applicant's T-Diet® range of products are especially important.

US/2003/0104033, for example, describes liquid formulations for enteral administration consisting of stabilized proteins and caseinates, further to other components to enhance preparation creaminess. In these formulations vegetal origin proteins are derived from soy. In the above mentioned document, protein efficiency rates of the protein mixture used are not mentioned. Numerous articles from the literature state the requirements for indispensable amino acids in adult humans, in all the cases regarding traditional oral diets (see for example Mahan LK, Stump SE (eds.) "Krause's food, nutrition and diet therapy", 11th Ed. Saunders, Philadelphia, Pennsylvania). In order to evaluate the characteristics of the potential enteral diets to be supplied, the most important factor to take into consideration is protein content since, as it is well-known, proteins are indispensable for maintaining body mass and cell functionality. Caloric density determines, besides calorie content, the density of the different nutrients comprised in the diet, especially liquid content.

EP 626175 A2 describes a liquid composition for enteral administration that contains soy protein hydrolysates.

The protein mixture described in this invention has very high protein quality indices (protein efficiency rates) and a high digestibility thus making the protein mixture of the invention more easily converted as compared with highly digestible proteins per excellence.

According to the protein content, products intended for enteral nutrition can be classified as normoproteic, hyperproteic and special. In the first case protein proportion is similar to that of a balanced diet, amounting to 12-18% of the total caloric content and with a calories to nitrogen ratio of about 120-150 kcal non-protein calories/g of nitrogen. In the second case, protein proportion is higher than 18%, with a calorie to nitrogen ratio lower than 120. Special products are those that are voluntarily unbalanced to be adapted to specific metabolic situations.

Therefore, the object of this invention is a novel protein mixture based on caseinate, pea protein and milk serum proteins enriched with glycomacropeptides

The protein mixture of the invention contains caseinate (50%), pea protein (25%) and milk serum protein (25%), the later enriched with glycomacropeptide. This new protein mixture based on caseinate, pea protein and milk serum proteins enriched with glycomacropeptide has a unique amino acid composition, very similar to that of the protein considered nutritionally as standard reference proteins (egg proteins). Its amino acid profile is shown below in Table 1:

**Table 1**

| Amino acid profile | per each 100 g of protein |
|---|---|
|  |  |
| Alanine ALA | 4.02 |
| Arginine ARG | 4.92 |
| Aspartic acid ASP | 9.20 |
| Cystine CYS | 1.05 |

(continued)

| Amino acid profile | per each 100 g of protein |
|---|---|
| Glutamic acid GLU | 22.68 |
| Glycine GLY | 2.50 |
| Histidine HIS | 2.52 |
| Isoleucine ILE | 5.52 |
| Leucine LEU | 9.67 |
| Lysine LYS | 8.32 |
| Methionine MET | 2.47 |
| Phenylalanine PHE | 4.87 |
| Proline PRO | 9.09 |
| Serine SER | 5.27 |
| Threonine THR | 4.85 |
| Tryptophane TRP | 1.32 |
| Tyrosine TYR | 4.57 |
| Valine VAL | 6.10 |
| MET+Cystein | 3.52 |
| PHE+Tyrosine | 9.44 |

The protein mixture of the invention shows that its growth coefficient efficiency and other biologic quality parameters are much higher than those of the standard protein used as reference in biological assays as evidenced by the exemplary embodiment described below.

This novel protein mixture provides amino acid nitrogen in enough quantity and quality and likewise, since glycomacropeptide is incorporated as well as sialic acid (N-acetylneuraminic acid), the administration of the food product of the invention has the advantage of its prebiotic nature, modulating intestinal macrobiota by means of increasing the number of bifidobacteria. Bifidobacteria proliferation has numberless effects, among them decreasing intestinal pH reducing or even inhibiting the growth of harmful bacteria, activating peristaltic movements and the immune system.

The product of this invention is described in further detail in the following exemplary embodiment.

Evaluation of the nutritive quality of the protein mixture of the invention and the methods to prepare its normoproteic and hyperproteic embodiments

The protein used in this exemplary embodiment is a combination of animal protein (75%) and vegetal protein (25%), more specifically 50% of caseinate, 25% of milk serum proteins and 25% of pea protein.

Method

*Experimental design*

The study was carried out in two phases, the first one in the Physiology Laboratory of the Pharmacy School of the University of Granada and the second one in the Animal Facilities of the same institution. In the first phase, the protein mixture of the invention was analyzed and in the second phase a product already produced at two different protein concentrations (normoproteic and hyperproteic), freeze-dried and adapted to meet rat nutrient requirements, was analysed.

Each experiment lasted 10 days, 3 days to adapt the animals to the diet and environmental conditions and 7 days to quantify solid intake, changes in weight and faeces and urine collection.

*Materials and Methods*

*Animals:*

Twenty 21-day-old *Wistar* rats, 10 males and 10 females were used in each experiment. The rats supplied by the Granada University Animal Facilities, with an average weight of between 50 g and 52 g were allocated into two groups having 5 females and 5 males each, .

The rats were placed in individual metabolic cages in a room at 23°C and with a light-darkness photoperiod of 12 hours. The diet and water were supplied daily *ad libitum.*

The diet was prepared at the Granada University Animal facilities pursuant to the recommendations of the American Institute of Nutrition (AIN), based on AIN-93G diet (Reeves, PG 1997) and making any necessary change when any nutrient was modified to adjust it to the rats' requirements.

Control and experimental diet composition in the first experiment

| Components | Diets (%) | |
|---|---|---|
| | Control | Experimental |
| Protein | 12 (casein) | 12* |
| Corn starch | 47.75 | 47.75 |
| Dextrinated starch | 13.2 | 13.2 |
| Sucrose | 10 | 10 |
| Soy oil | 7 | 7 |
| Cellulose (fibre) | 5 | 5 |
| AIN-93G-MX mineral supplement | 3.5 | 3.5 |
| AIN-93-VX vitamin supplement | 1 | 1 |
| L-cystine | 0.18 | 0.18 |
| Choline bitartrate | 0.25 | 0.25 |
| Antioxidant (mg) | 0.0014 | 0.0014 |
| *Assayed protein: 50% Potassium caseinate, 25% milk serum proteins and 25% pea protein. Components of diets adapted to meet the rats' requirements with freeze-dried normoproteic and hyperproteic enteral nutrition products | | |

| Components | Diets (%) | |
|---|---|---|
| | Normoproteic | Hyperproteic |
| Freeze-dried product | 68.97 | 56.07 |
| Corn starch | 11.10 | 24 |
| Sucrose | 10 | 10 |
| Cellulose (fibre) | 5 | 5 |
| AIN-93G-MX mineral supplement | 3.5 | 3.5 |
| AIN-93-VX vitamin supplement | 1 | 1 |
| L-cystine | 0.18 | 0.18 |
| Choline bitartrate | 0.25 | 0.25 |
| Antioxidant (mg) | 0.0014 | 0.0014 |

*Sample treatment:*

Collected faeces were frozen at -20°C, and then they were freeze-dried and ground. Urine was collected in a 5% HCl solution with Xiro-Taxiro indicator to avoid nitrogen losses.

*Analysis technique:*

Nitrogen determination was carried out by Kjeldahl method, using 0.5 g of dried, ground faeces, 5 mL of urine and 0.5 g of diet.

*Calculated Indices:*

Protein Efficiency Ratio (PER): This index is used to determine protein quality in accordance with animal weight gain.

$$PER = \frac{\text{Weight gain}}{\text{Protein intake}}$$

Apparent Digestibility Coefficient (ADC): Digestibility is a measure of food nutritive value, its ease of conversion into useful nutrients by the digestive tract. Apparent digestibility does not include excretion of endogenous origin faeces.

$$CDA = \frac{NI - NHe}{NI} \times 100$$

Percentage of Retention R/A: Retention indicates the metabolic use of amino acids and it is apparent whenever the nutrient fraction of endogenous origin is not taken into account. It is the ratio between retained and absorbed protein.

$$R/A = \frac{\text{Retained}}{\text{Absorbed}} \times 100$$

$$R/A = \frac{(NI - NHe) - (NOr)}{(NI - NHe)} \times 100$$

Retained Intake Ratio: Evidences if the protein intake has been incorporated into the human body; it is the retained protein intake that is used.

$$RI = \frac{\text{Retained}}{\text{Intake}} \times 100$$

$$RI = \frac{(NI - NHe) - (NOr)}{NI} \times 100$$

Results

*Growth, weight variation and food intake:*

Animal weight at the beginning and at the end of the first experiment was 58.52 g and 108.53 g for the experimental protein group and 56.30 g and 108.37 g for the control group respectively. In the second experiment, normoproteic and hyperproteic groups evidenced weight gains as from 42.30 g and 41.30 g, to 93.5 g and 92.40 g, respectively.

*Studied Indices:*

The following table shows a summary of the indices calculated to measure the quality of the protein mixture under study.

Protein Efficiency Ratio (PER), Apparent Digestibility Coefficient (ADC), Percentage of Retention (R/A) and Retained/Ingested Ratio (R/I) of the four groups under study

| Group | PER | ADC (%) | % R/A: | % R/I |
|---|---|---|---|---|
| Control Experimental | $3.88 \pm 0.61$ | $93.8 \pm 1.15$ | $84.00 \pm 3.93$ | $78.82 \pm 4.41$ |
| Protein | $4.04 \pm 0.29$ | $93.91 \pm 0.69$ | $85.33 \pm 4.51$ | $80.14 \pm 4.53$ |
| Normoproteic | $3.35 \pm 0.53$ | $90.63 \pm 0.77$ | $76.13 \pm 2.54$ | $68.99 \pm 2.03$ |
| Hyperproteic | $2.85 \pm 0.42$ | $91.22 \pm 0.68$ | $75.21 \pm 4.94$ | $68.63 \pm 4.82$ |

Note: average $\pm$ standard deviation.

It is to be noted that all PER values are above 2.5 and, from higher to lower ranking, we find the experimental group, the control group, the normoproteic group and the hyperproteic group. The European Society of Pediatric Gastroenterology, Hepatology and Nutrition established a PER value of 2.55 as the minimum nutritional requirement for infant milk formulas and, by way of similarity, for enteral nutrition diets. As it can be clearly seen, apparent digestibility coefficient of the protein mixture of the invention (the experimental group) has the highest value, 93.91%, very similar to that of the control group, 93.8%. Hyperproteic and normoproteic group values are 91.22% and 90.63% respectively. In the case of the Percentage of Retention and the Retained/Ingested Protein Ratio, their behaviour is very similar. Experimental and control groups are very similar and their values are higher than hyperproteic and normoproteic groups, which in turn show very similar values between them.

Discussion

*Growth, weight gain and food intake:*

Animal growth was increased in the four groups under study during the experiment, however, growth rate decreased during the last two days, without affecting their normal development. The Group with the highest weight gain was the experimental group, thus indicating that the diet used is adequate as compared with that of the control Group and its quality is good to maintain animal growth rate.

Food intake was clearly reflected in growth.

*Analyzed Indices:*

The experimental protein mixture had the highest PER calculated by us. In similar studies, Proll J. et al. in 1998 and Ghulan S. in 1997 reported a high PER value for casein, but lower than that obtained by us for the protein mixture of the invention. Van Dael P. et al. (2005) and Silva et al. (2003) had a PER value for casein lower than that reported by the above mentioned authors and lower than that found by us for the group with the lowest PER value (hyperproteic group).

It is worth noting that nitrogen consumption values as compared between the control group and the experimental group had little variations; however, PER values for the experimental group were much higher, thus evidencing the better quality of the protein mixture.

Millward DJ and Jackson AA (2003) found that digestibility of egg, milk, meat and soy isolated protein is higher than 95%, and digestibility of cereals, peas and rice is between 80% and 90%. The values found by us are 93.8% for the control diet (casein) and 93.91% for the protein mixture of the invention (experimental protein). Proll J et al. (1998) reported an apparent digestibility for casein of 91.2% (lower than the values reported by us for the experimental protein) and a true digestibility of 98.5%. Van Dael P et al. (2005) and Silva et al. reported a true digestibility for casein of 97% and 99.19% respectively.

Having found apparent digestibility values of about 94% and knowing that upon making corrections for true digestibility those values are increased, we can state that the ease of conversion of the protein under study is very good as compared to highly digestible proteins per excellence.

Normoproteic and hyperproteic diets had digestibility values lower than those of the groups mentioned above, probably due to the losses suffered on account of thermal treatment, but they were always above 90%

Percentage of retention (RP) and retained/ingested ratio (R/I) values are apparent values since endogenous protein values are not considered. However, some findings regarding Biologic Values (BV) and Net Protein Use (NPU) values are equivalent to those but they are true values and not apparent.

RP and R/I values reported by us for the experimental protein are higher than those reported by Van Dael P et al. in 2005, for casein Biologic Value and NPU.

In a publication issued by the FAO (1981) including several assays, an average BV of 79.7% and an average NPU of 72.1% for casein were reported; those values were lower than those found by us for the experimental protein and slightly higher than those of normo- and hyperproteic diets Therefore we can assume that the efficacy of the experimental protein ingested and used by the body is very high and adequate for growth and development.

It is worth noting that control and experimental diets are diets specially designed for rats containing all components and a palatability accepted by these animals. Normoproteic and hyperproteic diets are intended for human use and have been adapted to cover the nutritional requirements of these animals. However, they have undergone technical processes (mixing, thermal treatment and freeze-drying) that modify food organoleptic characteristics above all, and thus performance of indices calculated may not have been as good as that of the experimental group in spite of having the same protein mixture Therefore, we may conclude that the quality of the protein mixture of the invention is excellent and adequate for enteral or oral nutrition.

## Claims

1. A protein mixture in a food product intended for enteral or oral nutrition **characterized in that** it contains caseinate, pea protein and milk serum proteins and **in that** it has the following amino acid profile: Ala 4.02; Arg 4.92; Asp 9.20; Cys 1.05; Glu 22.68; Gly 2.50; His 2.52; Ile 5.52; Leu 9.67; Lis 8.32; Met 2.47; Phe 4.87; Pro 9.09; Ser 5.27; Thr 4.85; Trp 1.32; Tyr 4.57; Val 6.10; Met+cystein 3.52; Phe+tyrosine 9.44.

2. A protein mixture according to claim 1, **characterized in that** it contains 50% caseinate, 25% milk serum proteins and 25% of pea protein.

3. A protein mixture according to claim 1, **characterized in that** they are enriched with glycomacropeptide.

4. A protein mixture according to claim 1 **characterized in that** it has a protein efficacy ratio of 4.04±0.29.

5. A food product intended for enteral or oral nutrition **characterized in that** it includes a protein mixture according to Claim 1.

6. Use of a protein mixture according to claims 1-4 in a food product intended for enteral or oral nutrition.

## Patentansprüche

1. Eine Proteinmischung in einem für enterische oder orale Ernährung vorgesehenen Nahrungsmittel **dadurch gekennzeichnet, dass** sie Caseinat, Erbsenprotein und Molkeproteine enthält und dass sie folgendes Aminosäureprofil aufweist: Ala 4.02; Arg 4.92; Asp 9.20; Cys 1.05; Glu 22.68; Gly 2.50; His 2.52; Ile 5.52; Leu 9.67; Lis 8.32; Met 2.47; Phe 4.87; Pro 9.09; Ser 5.27; Thr 4.85; Trp 1.32; Tyr 4.57; Vat 6.10; Met+cystein 3.52; Phe+thyrosin 9.44.

2. Eine Proteinmischung gemäss Anspruch 1, **dadurch gekennzeichnet, dass** sie 50% Caseinat, 25 % Molkeprotein und 25% Erbsenprotein enthält.

3. Eine Proteinmischung gemäss Anspruch 1, **dadurch gekennzeichnet, dass** sie mit Glycomacroprotein angereichert ist.

4. Eine Proteinmischung gemäss Anspruch 1, **dadurch gekennzeichnet, dass** sie ein Proteineffizienzverhältnis von 4.04±0.29 aufweist.

5. Ein für enterische oder orale Ernährung vorgesehenes Nahrungsmittel **dadurch gekennzeichnet, dass** es eine Proteinmischung übereisntimmend mit Anspruch 1 enthält.

6. Gebrauch einer Proteinmischung gemäss Anspruch 1-4 in einem für enterische oder orale Ernährung vorgesehenen Nahrungsmittel.

**Revendications**

1. Un mélange de protéines dans un produit alimentaire destiné à la nutrition orale ou entérale **caractérisé en ce qu'**il contient de la caséinate, de la protéine de pois et des protéines de lactosérum, et **en ce qu'**il possède le profil suivant en acides aminés : Ala 4,02 ; Arg 4,92 ; Asp 9,20 ; Cys 1,05 ; Glu 22,68 ; Gly 2,50 ; His 2,52 ; Ile 5,52 ; Leu 9,67 ; Lis 8,32 ; Met 2,47 ; Phe 4,87 ; Pro 9,09 ; Ser 5,27 ; Thr 4,85 ; Trp 1,32 ; Tyr 4,57 ; Val 6,10 ; Met+cystéine 3,52 ; Phe+tyrosine 9,44.

2. Un mélange de protéines selon la revendication 1, **caractérisé en ce qu'**il contient 50 % de caséinate, 25 % de protéines de lactosérum et 25 % de protéines de pois.

3. Un mélange de protéines selon la revendication 1, **caractérisé en ce qu'**il est enrichi en glycomacropeptide.

4. Un mélange de protéines selon la revendication 1, **caractérisé en ce qu'**il possède un coefficient d'efficacité protéique de 4,04±0,29.

5. Un produit alimentaire destiné à la nutrition orale ou entérale **caractérisé en ce qu'**il contient un mélange de protéines selon la revendication 1.

6. Utilisation d'un mélange de protéines selon les revendications 1-4 dans un produit alimentaire pour la nutrition orale ou entérale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030104033 A

- EP 626175 A2

**Non-patent literature cited in the description**

- Krause's food, nutrition and diet therapy. Saunders